(19)

**Europäisches Patentamt**
**European Patent Office**
**Office européen des brevets**

(11) **EP 4 245 773 A1**

(12) **EUROPEAN PATENT APPLICATION**

(43) Date of publication:
**20.09.2023  Bulletin 2023/38**

(21) Application number: **22190672.0**

(22) Date of filing: **17.08.2022**

(51) International Patent Classification (IPC):
*C07K 16/28* (2006.01)      *A61K 39/395* (2006.01)
*A61K 38/39* (2006.01)      *A61K 39/12* (2006.01)
*A61K 48/00* (2006.01)      *A61P 31/20* (2006.01)
*A61P 35/00* (2006.01)

(52) Cooperative Patent Classification (CPC):
(C-Sets available)
**A61K 39/3955; A61K 35/761; A61K 38/39;**
**A61K 39/12; A61P 35/00; C07K 14/78;**
**C07K 16/2818; C12N 15/86;** A61K 2039/54;
A61K 2039/545; A61K 2300/00; C12N 2710/10343

(Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **18.03.2022  CN 202210270466**

(71) Applicant: **Guangzhou Doublle Bioproduct Co.,**
**Ltd.**
**Guangzhou Guangdong (CN)**

(72) Inventors:
• **Shuo, Tian**
  **Guangzhou (CN)**
• **Marilyn, Xiaohong Zhou**
  **Guangzhou (CN)**
• **Jianhua, Chen**
  **Guangzhou (CN)**
• **Wenlin, Huang**
  **Guangzhou (CN)**

(74) Representative: **Zaboliene, Reda**
**Metida**
**Business center Vertas**
**Gyneju str. 16**
**01109 Vilnius (LT)**

(54) **COMBINATION TREATMENT METHOD WITH E10A AND PD1 MONOCLONAL ANTIBODY FOR TUMOR AND PHARMACEUTICAL APPLICATION**

(57)    The present disclosure provides a cancer treatment method by using the combination of E10A and PD1 monoclonal antibody. This present disclosure adopts the combined application of recombinant human endostatin adenovirus and PD1 antibody, and it is found that it has a synergistic effect. The combined application can obviously inhibit the growth and proliferation of tumors, and has a good tumor inhibition rate, which is for the development of new tumor-targeted medicines.

EP 4 245 773 A1

(52) Cooperative Patent Classification (CPC): (Cont.)

C-Sets
**A61K 35/761, A61K 2300/00**

**Description**

**TECHNICAL FIELD**

The present disclosure belongs to the field of biomedicine, and particularly relates to a combination treatment method with E10A and PD1 monoclonal antibody for tumor and pharmaceutical application.

**BACKGROUND**

Tumors refer to new organisms formed by the proliferation of local tissue cells under the action of various tumorigenic factors, because these new organisms are mostly space-occupying massive protrusions, also named as neoplasms. Base on the cell characteristics of new organisms and the degree of harm to the body, tumors are classified into two categories: benign tumors and malignant tumors. Malignant tumors can be classified into carcinomas and sarcomas. Carcinomas refer to malignant tumors originating from epithelial tissue; sarcoma refers to malignant tumors that occur in mesenchymal tissues, including fibrous connective tissue, fat, muscle, blood vessels, bone and cartilage tissue.

PD-1 (programmed death receptor 1), also known as CD279 (cluster of differentiation 279), is an important immunosuppressive molecule that belongs to the immunoglobulin superfamily and is a membrane protein of 288 amino acid residues. PD-1 is an immune checkpoint that protects against autoimmunity mainly through two mechanisms, promoting apoptosis of antigen-specific T cells in lymph nodes (programmed cell death) and reducing the apoptosis of the production of regulatory T cells (anti-inflammatory, suppressor T cells). Therefore, immunomodulation of targeting PD-1 is of great significance for anti-tumor, anti-infection, anti-autoimmune diseases and organ transplantation survival. Its ligand PD-L1 can also be used as a target, and the corresponding antibody can also play the same role.

The ligand PD-L1 of PD1 is highly expressed in several cancers, and PD-1 binds to PD-L1, which can transmit inhibitory signals and reduce the proliferation of CD8+ T cells in lymph nodes, and PD-1 can also regulate Bcl-2 gene, which controls the accumulation of antigen-specific T cells in lymph nodes. Therefore, it is possible to develop monoclonal antibodies targeting PD-1 that enhance the immune system for the treatment of cancer, such as Nivolumab, Pidilizumab, Pembrolizumab and other monoclonal antibodies. Another study found that using a combination of anti-PDI and anti-CTLA4 antibodies was more effective in treating cancer than either antibody alone. Therefore, combination therapy has been shown to be more effective in the treatment of certain tumors. In addition, application CN201510492057.2 discloses the use of cyclic dinucleotide cGAMP combined with PD1 antibody Nivolumab in anti-tumor combination medication, which has significant inhibition on adenocarcinoma, lung cancer, breast cancer, melanoma, colon cancer and other cells. Application CN202110200473.6 discloses that cisplatin nanomedicines and PD1/PD-L1 inhibitors can be used in combination for tumor treatment. It is found that cisplatin nanomedicines can induce high expression of PD-L1 in tumors in a dose- and time-dependent manner. High expression of PD-L1 in tumors can be continuously induced after intravenous administration, so that PD1/PD-L1 inhibitors have more probability to block PD1/PD-L1 signaling pathway and activate killer T cells. In the combined use of PD1/PD-L1 inhibitors, the two can play a synergistic effect to further enhance the inhibition of tumors.

Endostatin is an angiogenesis inhibitor that can be applied to the principle of "starvation therapy" and is widely used in the treatment of various solid tumors such as lung cancer, liver cancer, pancreatic cancer, bowel cancer and prostate cancer. In the prior art, there is no report on the combined use of human endostatin and PD1 antibody in the treatment of tumors.

**SUMMARY**

In view of the above deficiencies, the present disclosure provides a combinatorial medicine for tumor treatment. The disclosure adopts human endostatin adenovirus and PD1 antibody in combination, and it is found that it can obviously inhibit the growth and development of tumors, has a better anti-tumor effect, and provides a new approach and method for tumor treatment. In order to realize the above-mentioned purpose of the disclosure, the technical scheme of the present disclosure is as follows:

In the first aspect, the present disclosure provides an application of a combination of human endostatin adenovirus and PD1 antibody in preparation of antitumor medicines.

Preferably the human endostatin adenovirus is administered at $5\times10^9$ vp/kg to $1000\times10^9$ vp/kg every time, preferably $1\times10^{10}$ vp/kg to $80\times10^{10}$ vp/kg, more preferably $2\times10^{10}$ vp/kg to $50\times10^{10}$ vp/kg.

Further preferably, the human endostatin adenovirus is administered 1-2 times a week, preferably once a week.

Preferably, the PD1 antibody comprises, but is not limited to, tislelizumab, nivolumab, palivizumab, atezolizumab, durvalumab, avelumab or toripalizumab etc., preferably toripalimab.

More preferably, the toripalimab is administered at 1 to 100 mg/kg, preferably 3 to 20 mg/kg, more preferably 5 to 10 mg/kg every time.

More preferably, the toripalimab is administered 1 to 3 times a week, preferably 2 times a week.

Preferably, the human endostatin adenovirus and the PD1 antibody are administered separately.

Preferably, the human endostatin adenovirus and PD1 antibody are administered simultaneously or sequentially.

Preferably, the human endostatin adenovirus and PD1 antibody are administered by intratumoral injection, intravenous injection or intraperitoneal injection.

Preferably, the tumor is a solid tumor.

More preferably, the tumors comprise but are not limited to: melanoma, ovarian cancer, breast cancer, cervical cancer, non-small cell lung cancer, thyroid cancer, liver cancer, esophageal cancer, lung cancer, prostate cancer, bladder cancer, glioblastoma tumor, gastric cancer, colon cancer, kidney cancer, etc.

More preferably, the tumor is colon cancer.

In a second aspect, the present disclosure provides an anti-tumor medicine, which comprises the above-mentioned human endostatin adenovirus and PD1 antibody.

Preferably, the human endostatin adenovirus is administered at $5 \times 10^9$ vp/k to $1000 \times 10^9$ vp/kg every time, preferably $1 \times 10^9$ vp/k to $80 \times 10^{10}$ vp/kg, more preferably $2 \times 10^9$ vp/k to $50 \times 10^{10}$ vp/kg.

More preferably, the human endostatin adenovirus is administered 1-2 times a week, preferably once a week.

Preferably, the PD1 antibody comprises, but is not limited to, tislelizumab, nivolumab, palivizumab, atezolizumab, durvalumab, avelumab or toripalizumab etc., preferably toripalimab.

More preferably, the toripalimab is administered at a dose of 1 to 100 mg/kg, preferably 3 to 20 mg/kg, more preferably 5 to 10 mg/kg each time.

More preferably, the toripalimab is administered 1 to3 times a week, preferably 2 times a week.

Preferably, the human endostatin adenovirus and the PD1 antibody are administered separately.

Preferably, the human endostatin adenovirus and PD1 antibody are administered simultaneously or sequentially.

Preferably, the human endostatin adenovirus and PD1 antibody are administered by intratumoral injection, intravenous injection or intraperitoneal injection.

Preferably, the tumor is a solid tumor.

More preferably, the tumors include but are not limited to: melanoma, ovarian cancer, breast cancer, cervical cancer, non-small cell lung cancer, thyroid cancer, liver cancer, esophageal cancer, lung cancer, prostate cancer, bladder cancer, glioblastoma tumor, gastric cancer, colon cancer, kidney cancer, etc.

More preferably, the tumor is colon cancer.

Preferably, the medicine also comprises a pharmaceutically acceptable carrier, which is selected from a group consisting of sustained release agent, excipient, filler, binder, wetting agent, disintegrating agent, absorption enhancer, and adsorption carrier, surfactants, lubricants and a mixture thereof.

In a further aspect, the present disclosure also provides a method for tumor treatment. The method comprising: administering to a subject a combination of an effective dosage of human endostatin adenovirus and an effective dossage of a PD1 antibody.

Preferably, the human endostatin adenovirus is administered at $5 \times 10^9$ vp/kg to $1000 \times 10^9$ vp/kg every time, preferably $1 \times 10^{10}$ vp/kg to $80 \times 10^{10}$ vp/kg, more preferably $2 \times 10^{10}$ vp/kg to $50 \times 10^{10}$ vp/kg.

More preferably, the human endostatin adenovirus is administered 1-2 times a week, preferably once a week.

Preferably, the PD1 antibody comprises, but is not limited to, tislelizumab, nivolumab, palivizumab, atezolizumab, durvalumab, avelumab or toripalizumab etc., preferably toripalimab.

More preferably, the toripalimab is administered at 1 to 100 mg/kg, preferably 3 to 20 mg/kg, more preferably 5 to 10 mg/kg every time.

More preferably, the toripalimab is administered 1 to 3 times a week, preferably 2 times a week.

Preferably, the human endostatin adenovirus and the PD1 antibody are administered separately.

Preferably, the human endostatin adenovirus and PD1 antibody are administered simultaneously or sequentially.

Preferably, the human endostatin adenovirus and PD1 antibody are administered by intratumoral injection, intravenous injection or intraperitoneal injection.

Preferably, the tumor is a solid tumor.

More preferably, the tumors comprise but are not limited to: melanoma, ovarian cancer, breast cancer, cervical cancer, non-small cell lung cancer, thyroid cancer, liver cancer, esophageal cancer, lung cancer, prostate cancer, bladder cancer, glioblastoma tumor, gastric cancer, colon cancer, kidney cancer, etc.

More preferably, the tumor is colon cancer.

Compared with the current available treatments, the positive and beneficial effects of the present disclosure are:

In this present disclosure, human endostatin adenovirus combined with PD1 antibody is used as a combined medicine, and it is found that it has a synergistic effect, and can significantly inhibit the growth and proliferation of tumors, and has a good tumor inhibition rate, which provides a reference for developing new and effective treatments for tumor.

## BRIEF DESCRIPTION OF THE DRAWINGS

Figure 1 shows the results of weight changes in mice.
Figure 2 shows tumor growth curve of colon cancer CT26 xenograft tumor.
Figure 3 shows the tumor weight test results.

## DETAILED DESCRIPTION OF THE EMBODIMENTS

The present disclosure will be described further in detail below with reference to specific embodiments. The following embodiments are not intended to limit the present disclosure, but are merely used to illustrate the present disclosure. The experimental methods used in the following examples, unless otherwise specified, the experimental methods without specific conditions in the examples are generally in accordance with conventional conditions, and the materials, reagents, etc. used in the following examples, are all commercially available, unless otherwise specified.

1.Human endostatin adenovirus

The human endostatin adenovirus described in the present disclosure carries the recombinantly constructed human endostatin gene, which can express human endostatin after transfection into tumor cells, inhibit the proliferation of vascular endothelial cells, inhibit tumor angiogenesis, and block the blood supply of tumor tissue, thereby specifically inhibiting tumor growth and inducing tumor cell apoptosis.

In the following examples, the human endostatin adenovirus of the present disclosure was manufactured by Guangzhou Doublle Biological Products Co., Ltd., the batch number is A20210501, and the product code is E10A.

The physicochemical properties and biological properties of the human endostatin adenovirus described in the present disclosure are as follows:

- Appearance: It is a colorless and clear liquid after being melted to room temperature;
- Strength (concentration): $1 \times 10^{12}$ vp/mL;
- Specification: 1mL/bottle;
- Purity: > 95%;
- Solvent and Solubility: Aqueous solution.

The human endostatin adenovirus of the present disclosure is administered at a dose of $5 \times 10^9$ vp/kg to $1000 \times 10^9$ vp/kg, preferably $1 \times 10^{10}$ vp/kg to $80 \times 10^{10}$ vp/kg, more preferably $2 \times 10^{10}$ vp/kg to $50 \times 10^{10}$ vp/kg. The human endostatin adenovirus of the present disclosure is administered 1 to 2 times a week, preferably once a week.

2.PD1 Antibody

PD1 is mainly expressed in activated T cells and B cells, and its function is to inhibit cell activation, which is a normal homeostatic mechanism of the immune system. Excessive T/B cell activation can cause autoimmune diseases. However, the tumor microenvironment will induce infiltrating T cells to overexpress PD1 molecules, and tumor cells will overexpress PD1 ligands PD-L1 and PD-L2, resulting in continuous activation of the PD1 pathway in the tumor microenvironment, inhibiting T cell function and unable to kill tumor cells. Antibodies to PD1 can block this pathway and partially restore the function of T cells, allowing these cells to continue killing tumor cells.

The PD1 antibody used in the present disclosure refers to a substance that can directly or indirectly neutralize, block, inhibit, reduce or hinder the activity of PD1. PD1 antibodies can neutralize, block, inhibit, reduce or hinder the activity of PD1 by binding to one or more PD1s. PD1 antibodies include: anti-PDI antibodies and antigen-binding fragments thereof, receptor molecules, and derivatives that specifically bind to PD1 to block its binding to one or more receptors, and the like. The PD1 antibody of the present disclosure includes, but is not limited to, tislelizumab, nivolumab, palivizumab, atezolizumab, durvalumab, avelumab or toripalizumab, etc. The preferred is toripalimab.

In the following examples, the PD1 antibody (toripalimab) of the present disclosure was purchased from Suzhou Zhonghe Biomedical Technology Co., Ltd., the batch number is 202103015, and the product code is PD1 monoclonal antibody or PD1.

The physicochemical properties and biological characteristics of toripalimab (PD1) used in the present disclosure are as follows:

- Appearance: colorless and clear liquid;
- Strength: 80 mg/2 mL,
- Specification: 1 bottle/box;
- Purity: >95%;
- Solvent and solubility: aqueous solution.

More specifically, PD1 is administered at 1 to 100 mg/kg, preferably 3 to 20 mg/kg, more preferably 5 to 10 mg/kg every time. More specifically, the toripalimab is administered 1 to 3 times a week, preferably 2 times a week.

3.Control

The reference substance of the present disclosure is the physiological saline solution (0.9% sodium chloride aqueous solution). In the following examples, the physiological saline solution (0.9% sodium chloride aqueous solution) of the present disclosure was purchased from Guangdong Yixiang Pharmaceutical Co., Ltd., and the batch number is 200816501.

The physicochemical properties and biological characteristics of the physiological saline solution (0.9% sodium chloride aqueous solution) of the present disclosure are as follows:

- Appearance: colorless and clear liquid;
- Components: sodium chloride, dilute hydrochloric acid, water for injection;
- Specification: 500mL:
- Strength: 4.5g sodium chloride

4.Medicines and Treatment Procedures

One embodiment of the present disclosure is a combinatorial medicine of human endostatin adenovirus and PD1 antibody for treating or preventing cancer.

The "medicine for treating or preventing cancer formed by combining human endostatin adenovirus and PD1 antibody" in the above scheme refers to the combination of human endostatin adenovirus and PD1 antibody in the treatment or prevention of cancer simultaneously or separately or sequentially administered. The medicament of the present disclosure can also be provided in the form of a formulation containing both human endostatin adenovirus and PD1 antibody. In addition, human endostatin adenovirus-containing medication and PD1 antibody-containing medication are provided separately, and these medications can be used simultaneously or sequentially.

In the present disclosure, simultaneous administration refers to the combined administration of human endostatin adenovirus and PD1 antibody at the same time period, which may be administered in the form of a formula, or may be administered in the form of a mixture prepared at the time of administration. Other dosage forms can also be administered at the same time. In the case of simultaneous administration, administration may be performed from separate routes or from the same route, and the administration or dosage forms may be the same or different from each other.

In the present disclosure, when the human endostatin adenovirus and the PD1 antibody are administered separately, the administration sequence of the human endostatin adenovirus and the PD1 antibody may be that the PD1 antibody is administered after the human endostatin adenovirus is administered, or human endostatin adenovirus and PD1 antibody may be administered simultaneously, or human endostatin adenovirus may be administered after PD1 antibody administration.

The sequential administration of human endostatin adenovirus and PD1 antibody in the present disclosure means that one medicine is administered and then another medicine is administered. The administration interval between human endostatin adenovirus and PD1 antibody is not particularly limited, and it can be considered factors such as route of administration or dosage form. Besides, in addition to factors such as administration route and dosage form, reference may also be made to the residual concentrations of the human endostatin adenovirus and PD1 antibody of the present disclosure in the subject. That is, when the PD1 antibody is administered before the administration of human endostatin adenovirus, the human endostatin adenovirus can be administered to the subject at the time point when the residual concentration of the PD1 antibody in the subject is detected and the desired effect of the human endostatin adenovirus is obtained. The concentration can be determined based on the analysis of the sample collected from the subject by various analytical methods known to those skilled in the art using separation devices such as chromatography, and vice versa.

Among the above-mentioned medicines, when human endostatin adenovirus and PD1 antibody are provided in separate preparations, the dosage forms of these medications may be the same dosage form or different dosage forms. For example, both medicines may be oral, non-oral, injections, drips, or intravenous drips in different dosage forms from each other; and both medicines may also be oral, non-oral, injections, drips, and intravenous drips in one and the same dosage form. In addition, one or more different medications may be combined in the above-mentioned medicines.

In the present disclosure, "subject" is not limited, but refers to a human or non-human mammal to which the medicine of the present disclosure is administered, such as mammals including cows, horses, dogs, sheep, and cats. Preferred subjects are humans. Subjects include patients (including humans and non-human mammals).

Example 1

1.Experimental Materials

(1) Experimental animals: hPD1-BALB/c mice, female/male, 4-6 weeks old, weighing 18-24 grams, provided by Jiangsu Jicui Yaokang Biotechnology Co., Ltd.
(2) Cell line: mouse colon cancer CT26 cell line, purchased from Wuhan Proceed Life Technology Co., Ltd., item number: CL-0071.
(3) RPMI-1640: Gibco.
(4) FBS: YOSHI.

.Experimental Method

(1) Modeling

On D0 day, 200 μL of a suspension containing $1\times10^6$ cells of colon cancer CT26 cell line was injected subcutaneously into the back of hPD1-BALB/c mice, one week later (D7), when the tumor grew to a diameter of (120-150) $mm^3$, the random grouping is shown in Table 1 below.

Table 1: Animal study plan

| Group | No. of mice | Name of Tested or Control | Dosage | Volume per dose (μL/mice) |
|---|---|---|---|---|
| A (control) | 10 | 0.9% saline solution | - | 50 |
| B (E10A) | 10 | Recombinant human endostatin adenovirus (E10A) | $1\times10^{10}$vp | 50 |
| C (PD1) | 10 | Toripalimab Injection (PD1) | 10mg/kg | 100 |
| D(E10A+PD1) | 10 | E10A + PD1 | $1\times10^{10}$vp+10mg/kg | 50+100 |

(2) Mode of administration and frequency of administration:

E10A is intratumoral injection, once a week, and its administration time points are D7, D14, D21;
PD1 mAb is intraperitoneal injection, twice a week, and its administration time points are D7, D10, D14, D17, D21, D24.

3) Determination of body weight:

The weight of mice was weighed with an electronic balance on D7, D14, D21, and D28.

(4) Detection of tumor growth rate:

D7, D10, D14, D17, D21, D24, D28 were used to measure the length and width of the tumor with a digital vernier caliper, and the tumor volume was calculated.

$$\text{Tumor Volume (mm}^3) = \text{width}^2 \times \text{length} \times 0.5$$

(5) Detection of tumor inhibition rate:

Mice were sacrificed on D28, and tumors were weighed.

$$\text{Tumor inhibition rate } (\%) = \frac{Wcontrol - Wtest}{Wcontrol} \times 100\%$$

(6) Statistical processing:

Statistical method: Student t test, P value < 0.05 means significant difference (two-sided).

3.Results

(1) Mice weight

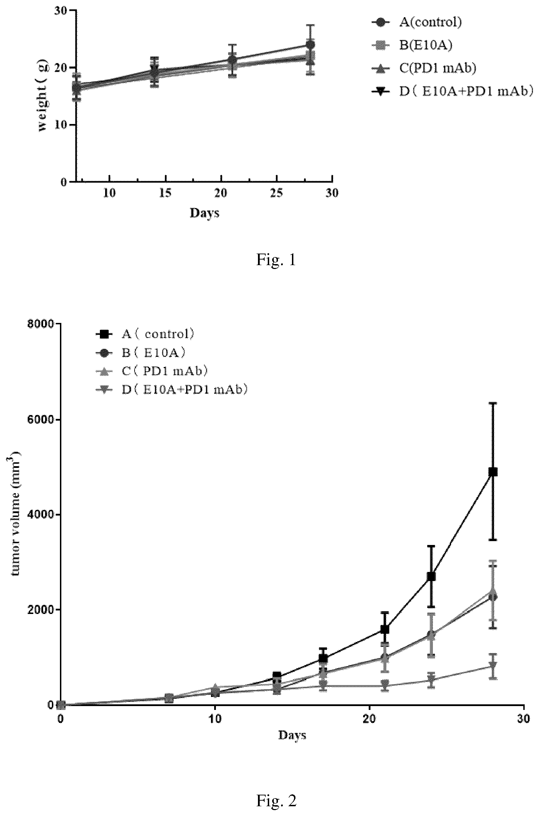

On D7, D14, D21, and D28, mice were weighed by electronic balance. There was no obvious change in body weight between the experimental group and the control group, indicating that there was no obvious systemic toxicity. See Table 2 and Figure 1 for details. Among them, 2 mice in group A died on day 28, which may be related to tumor progression.

Table 2: Mice Weight (X $\pm$ SD, g)

| Group | Experimental days | | | |
|---|---|---|---|---|
| | D7 | D14 | D21 | D28 |
| A (control) | 16.50±1.97 | 19.19±2.27 | 21.41±2.62 | 24.01±3.45 |
| B (E10A) | 16.18±2.18 | 18.85±2.06 | 20.51±2.08 | 22.16±2.79 |
| C (PD1) | 16.03±1.51 | 18.57±1.92 | 20.43±2.07 | 21.34±2.51 |
| D(E10A+PD1) | 16.54±2.10 | 19.66±2.12 | 20.50±1.93 | 21.67±2.79 |

(2) Tumor growth rate

The tumor volume in group B (group E10A) and group C (group C (PD1 monoclonal antibody) on D14, D17, D21, D24, and D28 was smaller than that in group A (normal saline group), but there was no statistical significance (P>0.05). The tumor volume of group D (E10A+PD1 monoclonal antibody group) on D14 was smaller than that of group A (normal saline group) but not statistically significant (P>0.05), and there was a significant difference between D17 and group A (normal saline group) (P < 0.05), there were extremely significant differences (P < 0.01) between D21, D24, D28 and group A (normal saline group) (P < 0.01). The data are shown in Table 3 and Figure 2.

Table 3: Tumor growth curve of colon cancer CT26 xenograft tumor (mm$^3$)

| Group | Experimental days | | | | | | |
|---|---|---|---|---|---|---|---|
| | D7 | D10 | D14 | D17 | D21 | D24 | D28 |
| A (control) | 135.78 | 264.38 | 568.62 | 970.14 | 1588.87 | 2700.59 | 4901.55 |
| B (E10A) | 155.62 | 251.31 | 335.26 | 672.73 | 998.72 | 1483.55 | 2270.68 |
| C (PD1) | 163.67 | 380.30 | 444.15 | 649.07 | 972.07 | 1450.72 | 2410.05 |
| D(E10A+PD1) | 154.77 | 264.06 | 331.96 | 400.82* | 405.10** | 519.58** | 809.61** |
| Note: compared with Group A, * = P<0.05, ** = P<0.01 | | | | | | | |

(3) Tumor inhibition rate

The tumor weight of group B (E10A group) was 2.08 (n=10), the tumor weight of group C (PD1 mAb group) was 1.88 (n=10), and the tumor weight of group D (E10A+PD1 mAb group) was 0.69 (n=10), the tumor weight of group A (normal saline group) was 3.72 (n=8);

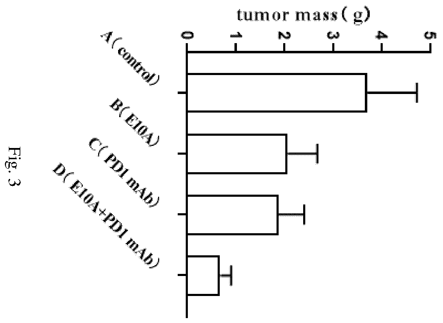

Tumor growth inhibition rate: group B (group E10A) vs group A (saline group) = 43.99% (P=0.168); group C (PD1 monoclonal antibody group) vs group A (saline group) = 49.43% (P= 0.111); group D (E10A+PD1 monoclonal antibody group) and group A (normal saline group) =81.41% (P=0.0005), indicating that the combination of E10A+PD1 monoclonal antibody group significantly inhibited the growth of colon cancer CT26 xenografts. The data are presented Table 4 and Figure 3.

Table 4: Inhibitory effect of E10A and PD1 mAbs on colon cancer CT26

| Group | No. of mice | Average Tumor Weight (g) | Tumor inhibition rate (%) |
|---|---|---|---|
| A (control) | 8 | 3.72 | - |
| B (E10A) | 10 | 2.08 | 43.99 |
| C (PD1) | 10 | 1.88 | 49.43 |
| D (E10A+PD1) | 10 | 0.69 | 81.41** |
| Note: compared with Group A, * = P<0.05, ** = P<0.01 | | | |

(4) Calculation of Two-drug interaction index (CDI)

Table 5: The ratio of tumor weight in each group to the control group

| Group | No. of mice | Average Tumor Weight (g) | Ratio to the control |
|---|---|---|---|
| A (control) | 8 | 3.72 | 1 |
| B (E10A) | 10 | 2.08 | 0.56 |
| C (PD1) | 10 | 1.88 | 0.51 |
| D (E10A+PD1) | 10 | 0.69 | 0.19 |

The two-drug interaction coefficient (CDI) was used to evaluate the nature of the two-drug interaction, and the CDI was calculated according to the following formula: CDI=AB/(A×B), where AB was the ratio of the tumor weight of the two-drug combination group to the control group, A or B is the ratio of tumor weight of each single drug group to that of the control group. CDI<1, the action properties of the two drugs are synergistic; CDI=1, the action properties of the two drugs are additive; CDI>1, the action properties of the two drugs are antagonistic.

According to Table 5, CDI=0.19/(0.56×0.51)=0.67<<1. Therefore, the synergistic effect of E10A and PD1 mAb is very significant.

The above-mentioned embodiments only represent several embodiments of the present disclosure, and the descriptions thereof are specific and detailed, but should not be regarded as a limitation on the scope of the patent of the present disclosure. It should be noted that various modifications and improvements can be made for those of ordinary skill in the art, without departing from the concept of the present disclosure, which shall fall into the protection scope of the present disclosure. Therefore, the protection scope of the patent of the present disclosure should be subject to the appended claims.

**Claims**

1. A method for treating tumors, wherein the method comprises: administering to a subject a combination of an effective dosage of human endostatin adenovirus and an effective dosage of a PD1 antibody.

2. The method according to claim 1, wherein the human endostatin adenovirus is administered at an amount of $5 \times 10^9$ vp/kg to $1000 \times 10^9$ vp/kg, preferably $1-80 \times 10^{10}$ vp/kg; more preferably $2 \times 10^{10}$ vp/kg to $50 \times 10^{10}$ vp/kg.

3. The method according to claim 1, wherein the PD1 antibody is selected from a group consisting of tislelizumab, nivolumab, panlizumab, atezolizumab, durvalizumab, avizumab Monoclonal antibody and toripalimab, preferably toripalimab.

4. The method according to claim 3, wherein the toripalimab is administered with 1 to 100 mg/kg every 1 time, preferably 3 to 20 mg/kg, more preferably 5 to 10 mg/kg.

5. The method according to claim 1, wherein the human endostatin adenovirus and the PD1 antibody are administered separately, simultaneously or sequentially.

6. The method according to claim 1, wherein the human endostatin adenovirus and PD1 antibody are administered by intratumoral injection, intravenous injection or intraperitoneal injection.

7. The method according to claim 1, wherein the tumor is a solid tumor.

8. The method according to claim 7, wherein the tumor is melanoma, ovarian cancer, breast cancer, cervical cancer, non-small cell lung cancer, thyroid cancer, liver cancer, esophageal cancer, lung cancer, prostate cancer, bladder cancer, glioblastoma, gastric cancer, colon cancer and/or kidney cancer, the tumor is colon cancer.

9. An anti-tumor medicine, wherein the medicine comprises human endostatin adenovirus and PD1 antibody.

10. The medicine according to claim 9, wherein the medicine further comprises a pharmaceutically acceptable carrier, and the carrier is selected from a group consisting of sustained release agent, excipient, filler, adhesive, wetting agent, disintegrant, absorption enhancer, adsorption carrier, surfactant or lubricant, or a mixture thereof.

Fig. 1

Fig. 2

Fig. 3

## EUROPEAN SEARCH REPORT

**Application Number**

EP 22 19 0672

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Luo Zhiguo: "Maintenance Treatment of Toripalimab(JS001) in Patients With Unresectable Locally Advanced or Metastatic Mucosal Melanoma – Full Text View – ClinicalTrials.gov", ClinicalTrials.gov, 15 July 2020 (2020-07-15), pages 1-7, XP093043936, Retrieved from the Internet: URL:https://clinicaltrials.gov/ct2/show/NCT04472806 [retrieved on 2023-05-03] * (p 2, first lines) *  ----- | 1-10 | INV. C07K16/28 A61K39/395 A61K38/39 A61K39/12 A61K48/00 A61P31/20 A61P35/00 |
| Y | LV WEIZE ET AL: "Safety and efficacy of nivolumab plus recombinant human endostatin in previously treated advanced non-small-cell lung cancer", TRANSLATIONAL LUNG CANCER RESEARCH, vol. 11, no. 2, 1 February 2022 (2022-02-01), pages 201-212, XP093043915, Hong Kong ISSN: 2218-6751, DOI: 10.21037/tlcr-22-49 * (p 202, col 2, l 2) *  ----- -/-- | 1-10 | |
| | | | **TECHNICAL FIELDS SEARCHED (IPC)** C07K A61K A61P C12N |

The present search report has been drawn up for all claims

| Place of search | Date of completion of the search | Examiner |
|---|---|---|
| Munich | 8 May 2023 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 6

Europäisches
Patentamt

European
Patent Office

Office européen
des brevets

## EUROPEAN SEARCH REPORT

Application Number

EP 22 19 0672

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LI-XIA LI ET AL: "Antitumor efficacy of a recombinant adenovirus encoding endostatin combined with an E1B55KD-deficient adenovirus in gastric cancer cells", JOURNAL OF TRANSLATIONAL MEDICINE, BIOMED CENTRAL, vol. 11, no. 1, 14 October 2013 (2013-10-14), page 257, XP021164416, ISSN: 1479-5876, DOI: 10.1186/1479-5876-11-257 (p 4, col 2, first lines); * abstract * | 1-10 | |
| Y | LI Q ET AL: "Inhibition of angiogenesis and the growth of murine colon cancer with recombinant human endostatin adenovirus ¦ Journal of Clinical Oncology", JOURNAL OF CLINICAL ONCOLOGY, vol. 26, no. 15, 20 May 2008 (2008-05-20), pages 14634-14634, XP093044098, * abstract * | 1-10 | |
| | | | TECHNICAL FIELDS SEARCHED (IPC) |

-/--

The present search report has been drawn up for all claims

| Place of search | Date of completion of the search | Examiner |
|---|---|---|
| Munich | 8 May 2023 | Bigot-Maucher, Cora |

EPO FORM 1503 03.82 (P04C01)

Europäisches
Patentamt

European
Patent Office

Office européen
des brevets

**EUROPEAN SEARCH REPORT**

Application Number

EP 22 19 0672

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ADHIM Z ET AL: "E10A, an adenovirus-carrying endostatin gene, dramatically increased the tumor drug concentration of metronomic chemotherapy with low-dose cisplatin in a xenograft mouse model for head and neck squamous-cell carcinoma", CANCER GENE THERAPY, NATURE PUBLISHING GROUP US, NEW YORK, vol. 19, no. 2, 25 November 2011 (2011-11-25), pages 144-152, XP037761587, ISSN: 0929-1903, DOI: 10.1038/CGT.2011.79 [retrieved on 2011-11-25] (p 146, col 1, para 1); * abstract * | 1-10 | |
| Y | CHEN JIANXIN ET AL: "Comparison of atezolizumab, durvalumab, pembrolizumab, and nivolumab as first-line treatment in patients with extensive-stage small cell lung cancer : A systematic review and network meta-analysis", MEDICINE, vol. 100, no. 15, 16 April 2021 (2021-04-16), page e25180, XP093044002, US ISSN: 0025-7974, DOI: 10.1097/MD.0000000000025180 * abstract * | 1-10 | **TECHNICAL FIELDS SEARCHED (IPC)** |

-/--

The present search report has been drawn up for all claims

| Place of search | Date of completion of the search | Examiner |
|---|---|---|
| Munich | 8 May 2023 | Bigot-Maucher, Cora |

EPO FORM 1503 03.82 (P04C01)

page 3 of 6

EP 4 245 773 A1

**EUROPEAN SEARCH REPORT**

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANG XIAOHUI ET AL: "Effectiveness and safety of PD-1/PD-L1 inhibitors in the treatment of solid tumors: a systematic review and meta-analysis", ONCOTARGET, vol. 8, no. 35, 29 August 2017 (2017-08-29), pages 59901-59914, XP093044067, DOI: 10.18632/oncotarget.18316 * table 1 * | 1-10 | |
| Y | WEI XIAO-LI ET AL: "A phase I study of toripalimab, an anti-PD-1 antibody, in patients with refractory malignant solid tumors", CANCER COMMUNICATIONS, vol. 40, no. 8, 1 August 2020 (2020-08-01), pages 345-354, XP093044123, ISSN: 2523-3548, DOI: 10.1002/cac2.12068 * (p 347, col 1, last para); table 2 * | 1-10 | |
| Y | WANG ZHIJIE ET AL: "A Phase 2 Study of Tislelizumab in Combination With Platinum-Based Chemotherapy as First-line Treatment for Advanced Lung Cancer in Chinese Patients", LUNG CANCER., vol. 147, 1 September 2020 (2020-09-01), pages 259-268, XP093044189, NL ISSN: 0169-5002, DOI: 10.1016/j.lungcan.2020.06.007 * abstract * | 1-10 | **TECHNICAL FIELDS SEARCHED (IPC)** |

-/--

The present search report has been drawn up for all claims

| Place of search | Date of completion of the search | Examiner |
|---|---|---|
| Munich | 8 May 2023 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 245 773 A1**

# EUROPEAN SEARCH REPORT

Application Number

EP 22 19 0672

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Hui Zhou: "A Randomized, Double-blind, Phase III Study Evaluating the Efficacy and Safety of Sintilimab or Placebo in Combination with Pemetrexed and Platinum-based Chemotherapy in the First-line Treatment of Advanced or Recurrent Non-squamous Non-small Cell Lung Cancer Study Phase: Phase III", Innovent Biologics, 9 August 2019 (2019-08-09), pages 1-133, XP093044492, Retrieved from the Internet: URL:https://clinicaltrials.gov/ProvidedDocs/39/NCT03607539/Prot_SAP_000.pdf [retrieved on 2023-05-04] * p 34, para 2 * | 1-10 | |
| A | HYUN TAE LEE ET AL: "Molecular mechanism of PD-1/PD-L1 blockade via anti-PD-L1 antibodies atezolizumab and durvalumab", SCIENTIFIC REPORTS, vol. 7, no. 1, 17 July 2017 (2017-07-17), pages 1-12, XP055454737, DOI: 10.1038/s41598-017-06002-8 * abstract * | 1-10 | |
| | | | TECHNICAL FIELDS SEARCHED (IPC) |
| A | COLLINS JULIE M. ET AL: "Product review: avelumab, an anti-PD-L1 antibody", HUMAN VACCINES & IMMUNOTHERAPEUTICS, vol. 15, no. 4, 20 December 2018 (2018-12-20), pages 891-908, XP055773567, US ISSN: 2164-5515, DOI: 10.1080/21645515.2018.1551671 * abstract * | 1-10 | |

-/--

The present search report has been drawn up for all claims

| Place of search | Date of completion of the search | Examiner |
|---|---|---|
| Munich | 8 May 2023 | Bigot-Maucher, Cora |

| | Europäisches Patentamt European Patent Office Office européen des brevets | **EUROPEAN SEARCH REPORT** | | Application Number EP 22 19 0672 |
|---|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | CN 115 120 744 A (UNIV SICHUAN) 30 September 2022 (2022-09-30) * the whole document * ----- | 1-10 | |

TECHNICAL FIELDS SEARCHED (IPC)

The present search report has been drawn up for all claims

| Place of search | Date of completion of the search | Examiner |
|---|---|---|
| Munich | 8 May 2023 | Bigot-Maucher, Cora |

## ANNEX TO THE EUROPEAN SEARCH REPORT
## ON EUROPEAN PATENT APPLICATION NO.

EP 22 19 0672

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2023

| Patent document cited in search report | | Publication date | Patent family member(s) | Publication date |
|---|---|---|---|---|
| CN 115120744 | A | 30-09-2022 | NONE | |

For more details about this annex : see Official Journal of the European Patent Office, No. 12/82

EPO FORM P0459

**EP 4 245 773 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201510492057
- CN 202110200473